(19) Europäisches Patentamt
European Patent Office
Office européen des brevets

(11) EP 0 744 393 B1

(12) EUROPEAN PATENT SPECIFICATION

(45) Date of publication and mention of the grant of the patent:
**16.08.2000 Bulletin 2000/33**

(51) Int Cl.[7]: **C07C 51/567**, C08F 4/00, C07F 9/535, C10M 159/12

(21) Application number: **96303528.2**

(22) Date of filing: **17.05.1996**

# (54) Process for the preparation of polyalkene-substituted succinic acylating agents in the presence of a dihelotriphenylphosphorane as catalysts

Verfahren zur Herstellung von Acylierungsmitteln auf Basis von Polyalken-substituierter Bernsteinsäure in Gegenwart von Dihalotriphenylphosphoran als Katalysator

Procédé de préparation d'agents d'acylation à base d'acide succinique substitué par polyalkylène en présence de dihalotriphénylephosphorane comme catalyseur

(84) Designated Contracting States:
**BE DE FR**

(30) Priority: **23.05.1995 GB 9510333**

(43) Date of publication of application:
**27.11.1996 Bulletin 1996/48**

(73) Proprietor: **ETHYL PETROLEUM ADDITIVES LIMITED**
**Bracknell, Berkshire RG12 2UW (GB)**

(72) Inventors:
- **Scattergood, Roger**
  **Reading, Berkshire, RG7 1DP (GB)**
- **Kerrison, Jacky**
  **Andover, Hampshire, SP11 7DA (GB)**

(74) Representative: **Cresswell, Thomas Anthony**
**J.A. KEMP & CO.**
**14 South Square**
**Gray's Inn**
**London WC1R 5LX (GB)**

(56) References cited:
**US-A- 3 954 812** **US-A- 3 960 900**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a process for preparing polyalkene-substituted succinic acylating agents, in particular to the catalysed preparation of polyisobutenyl succinic anhydride.

Polyalkene-substituted succinic acylating agents are of industrial significance as they are employed, for example, in the manufacture of ashless dispersants. The latter are widely used in hydrocarbonaceous liquids including fuels such as gasoline, diesel and jet fuel and in lubricating oil and functional fluid compositions such as automotive crank case lubricating oils, automatic transmission oils and gear oils.

Numerous techniques have been proposed for the preparation of polyalkene-substituted succinic acylating agents. Typically, these involve the reaction at elevated temperature of a polyalkene and an unsaturated aliphatic dicarboxylic acid or acid anhydride, for example maleic acid or anhydride. However, the reaction does not proceed cleanly and does not give a theoretical result. On the contrary, the yield of desired product is much less than expected. Also, the reaction often results in the production of an undesirable sludge and/or tarry residue which must be separated from the reaction product. The production of tarry residue is particularly troublesome as it can cause fouling of the reactor. It is believed that decomposition of the unsaturated dicarboxylic reactant under the reaction conditions employed is responsible for sludge and tar formation.

A number of techniques have been proposed for improving the yield of polyalkene-substituted succinic acylating agents obtained by such reactions. For example, USP 3,927,041 discloses the reaction of viscous polybutenes and unsaturated dicarboxylic acid anhydrides in the presence of brominated dialkylhydantoin (1,3-dibromo-5,5-dialkylhydantoin). Use of the latter is said to reduce the amount of undesirable by-products resulting from decomposition of the dicarboxylic acid anhydride reactant. However, the yield of desired product is not particularly acceptable and the amount of sludge generated still relatively high. The use of 1,3-dibromo-5,5-dialkylhydantoin is also known in which the dicarboxylic acid anhydride reactant is added to the polybutene very slowly.

USP 3,953,475, USP 3,954,812 and USP 3,960,900 respectively describe reacting polybutene and a dicarboxylic acid anhydride in the presence of an α-bromoketone (eg α-bromoacetophenone), N-bromosuccinimide and a substituted aliphatic hydrocarbon (e.g. tetrabromoethane and phenothiazine).

It has now been found that it is possible to prepare polyalkene-substituted succinic acylating agents by reacting the conventional reactants in the presence of a dihalotriphenylphosphorane catalyst. This has the advantage over the prior art techniques of giving the desired product in improved yield and/or results in a reduction in the amount of undesirable by-products formed, particularly the amount of tar.

In accordance with the present invention there is provided a process for the preparation of a polyalkene-substituted succinic acylating agent which comprises reacting a polyalkene and a compound of formula:

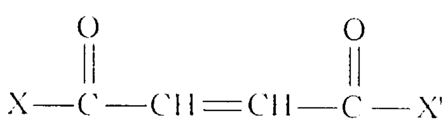

(I)

in which X and X' are the same or different and are each independently selected from -OH, -Cl or -OR" represents alkyl of 1 to 6 carbon atoms, or X and X' together form a group -O-, characterised in that the reaction takes place in the presence of a dihalotriphenylphosphorane.

The reaction is typically carried out in the presence of from 5 to 500, preferably 100 to 400, ppm of dihalotriphenylphosphorane. As the latter dichlorotriphenylphosphorane or dibromotriphenylphosphorane may be used. While dichlorotriphenylphosphorane is effective it is preferred however to use dibromotriphenylphosphorane. Dibromotriphenylphosphorane and dichlorotriphenylphosphorane are commercially available.

In the process of the invention the mole ratio of polyalkene to compound of formula (I) in the reaction is typically 1:5 to 1:0.8, generally 1:1.05 to 1:1.5. More favourable results are however obtained using a molar ratio of approximately 1:1.1.

The reaction is typically conducted at a temperature of from 150 to 300°C, preferably at a temperature of between 200 and 250°C. Usually the reaction is complete in 5 hours.

As described above, the process of the invention exhibits advantages over conventional preparative techniques. The present invention thus includes within its scope a method of improving the yield and/or reducing the amount of undesirable by-products formed in the preparation of a polyalkene-substituted succinic acylating agent, which comprises preparing said acylating agent by the process herein described.

In the compound of formula (I) at least one of X and X' is such that the polyalkene substituted acylating agent obtained can function as a carboxylic acylating agent. The ability to function as such is required for the preparation of ashless dispersants. In other words, at least one of X and X' must be such that the substituted acylating agent can

esterify alcohols, form amides or amine salts with ammonia or amines, form metal salts with reactive metals or basically reacting metal compounds, and otherwise function as a conventional carboxylic acid acylating agent. Transesterification and transamidation reactions are considered as conventional acylation reactions.

According to a feature of the invention the polyalkene is reacted with a compound of formula (I) in which X and X' are each independently selected from -OH, -Cl or -OR" in which R" represents alkyl of 1 to 6 carbon atoms, or X and X' together form a group -O-. Preferably, the polyalkene is reacted with maleic acid, maleic anhydride, fumaric acid, or a combination thereof. Of these the most preferred reactant is maleic anhydride.

Polyalkenes which may be used in the process of the present invention include those known in the art, for example from EP-A-460309 and USP 4,234,435. Preferably, the polyalkene used has a number average molecular weight of from about 500 to about 10,000, more preferably in the range of from about 700 to about 5,000. It is further preferred that the polyalkene is polyisobutene.

In preparing polyalkenes which may be used conventional techniques known to those skilled in the art may be employed. Typically these involve suitably controlling polymerisation temperatures, regulating the amount and type of polymerisation initiator and/or catalyst, employing chain terminating groups in the polymerisation procedure, and the like. Other conventional techniques such as stripping (including vacuum stripping) a very light end and/or oxidatively or mechanically degrading high molecular weight polyalkene to produce lower molecular weight polyalkenes can also be used.

It is particularly preferred to apply the process of the present invention to the preparation of polyisobutenyl succinic anhydride by the reaction of polyisobutene and maleic anhydride in the presence of dihalotriphenylphosphorane catalyst. Examples of polyisobutenes which may be used include the commercially available polyisobutenes Napvis 120 and Glissopal ES 3252. Napvis 120 is a polyisobutene having a molecular weight of 2100 available from BP Chemicals. Glissopal ES3252 is a high reactive polyisobutene having a molecular weight of 2300 and is available from BASF.

Commercially, the process of the present invention may be conducted in a batch wise or continuous manner in a stirred-tank type autoclave or equivalent reaction vessel provided intimate contact between the reactants. The operation of continuous and batch wise reactions of the kind which may be used are well known in the art.

The polyalkenyl-substituted succinic acylating agents prepared in accordance with the invention are particularly suitable for preparing ashless dispersants. Typically, this involves reaction of the acylating agent with a polyamine followed by optional post-treatment of the product obtained. Techniques for the preparation of ashless dispersant from the kind of acylating agents prepared in accordance with the present invention are well known in the art.

The following Examples illustrate the present invention.

## EXAMPLE 1

Preparation of Polyisobutenyl succinic anhydride

A flange flask equipped with stirrer, gas inlet tube and thermocouple was charged with polyisobutene. The polyisobutene was heated with stirring to a temperature of 235°C and the catalyst under test added. After mixing well for 20 minutes molten maleic anhydride was added over a period of 30 minutes using a pressure equalising dropping funnel. The reaction mixture was then stirred for 5 hours at 235°C under reflux conditions. At the end of this period the reaction mixture was stripped for 2 hours using a vacuum of approximately 13332.2 Pa (100 mmHg) and a slight sparge of nitrogen. A filter aid (Dicalite Speedplus) was added and after cooling to 180°C the product filtered.

The activity of the polyisobutenyl succinic anhydride (PIBSA) obtained was measured as a percentage using the method set out below.

A chromatographic column was prepared using silica gel (Davidson Grade 62, mesh size 60-200). The PIBSA was dissolved in hexane, placed on the column and eluted with hexane to separate off free polyisobutene. The eluent was then evaporated to dryness to give the weight of polyisobutene. The activity was calculated from the formula:

$$(\text{Sample Weight} - \text{Weight polyisobutene eluted}) \times 100/\text{Sample Weight}.$$

The amount of tar generated was measured using the method set out below.

The flange flask is weighed before the addition of polyisobutene and after the reaction all traces of product are removed using heptane. The flask is then dried (by standing overnight or using nitrogen) and the flask reweighed. The difference between the initial weight of the flask and the final weight gives an approximate value for the amount of tar produced. The value obtained is a measure only of the amount of tar residual on the apparatus. In practice, some tar may also be suspended in the final product itself.

The following table shows the amounts of the particular reactants used and the results obtained.

TABLE 1

| POLYISOBUTENE (PIB) USED | WEIGHT PIB (g) | WEIGHT MALEIC ANHYDRIDE (g) | CATALYST USED | AMOUNT OF CATALYST (ppm) | ACTIVITY (%) | TAR (g/kg) |
|---|---|---|---|---|---|---|
| NAPVIS 120 | 1000 | 50.70 | NONE | - | 42.8 | 6.2 |
| NAPVIS 120 | 1000 | 50.70 | DBH | 200 | 51.2 | - |
| NAPVIS 120 | 1000 | 50.70 | DBH | 210 | 51.4 | 1.7 |
| NAPVIS 120 | 1000 | 49.90 | NBS | 190 | 43.6 | 1.4 |
| NAPVIS 120 | 1000 | 50.70 | TBE | 200 | 50.8 | 1.2 |
| NAPVIS 120 | 1000 | 50.70 | PT | 200 | 47.1 | 1.1 |
| NAPVIS 120 | 1000 | 50.70 | NBS & PT | 80:110 | 53.7 | 2.1 |
| NAPVIS 120 | 1000 | 50.70 | TBE & PT | 100:100 | 45.0 | 2.0 |
| NAPVIS 120 | 1000 | 50.70 | DBH & PT | 110:110 | 52.6 | 1.3 |
| NAPVIS 120 | 1000 | 50.70 | DBTPP | 180 | 56.9 | 0.2 |
| NAPVIS 120 | 2000 | 101.40 | DBTPP | 200 | 59.8 | 0.6 |
| NAPVIS 120 | 1000 | 50.70 | DBTPP | 400 | 58.3 | 0.4 |
| NAPVIS 120 | 1000 | 50.80 | DCTPP | 200 | 46.7 | 2.6 |
| ES3252 | 1000 | 50.76 | None | - | 57.4 | 2.9 |
| ES3252 | 1000 | 57.35 | DBH | 200 | 62.7 | 1.4 |
| ES3252 | 1000 | 55.54 | DBTPP | 230 | 71.9 | 0.8 |

In the table:
DBH stands for 1,3-dibromo-5,5-dimethylhydantoin.
NBS stands for N-bromosuccinimide.
TBE stands for tetrabromoethane.
PT stands for phenothiazine.
DBTPP stands for dibromotriphenylphosphorane.
DCTPP stands for dichlorotriphenylphosphorane.

The amount of tar produced is represented in grams per kilogram of polyisobutene used.

As can be seem from this table using the process of the present invention leads to an increased conversion of polyisobutene to polyisobutenyl succinic anhydride and a significant reduction in the amount of tar generated compared with when no catalyst is used. The results obtained using dibromotriphenylphosphorane show that these advantages are also observed when compared with catalysts already used in the art.

## EXAMPLE 2

Autoclave preparation of polyisobutenyl succinic anhydride.

A two gallon Parr stainless steel autoclave (Model 4552) was cleaned using acetone/heptane and with light scrubbing with wire-wool. The autoclave was charged with polyisobutene together with the required charge of maleic anhydride and catalyst. The autoclave is sealed and evacuated to approximately 26664.4 Pa (200mmHg). The temperature of the autoclave was then raised according to the following schedule.

| TIME ELAPSED (MINUTES) | VACUUM PRESSURE (mmHg) | TEMPERATURE AT WHICH AUTOCLAVE SET (°C) | ACTUAL AUTOCLAVE TEMPERATURE (°C) |
|---|---|---|---|
| 0 | 460 | 150 | ~20 |

(continued)

| TIME ELAPSED (MINUTES) | VACUUM PRESSURE (mmHg) | TEMPERATURE AT WHICH AUTOCLAVE SET (°C) | ACTUAL AUTOCLAVE TEMPERATURE (°C) |
|---|---|---|---|
| 30 | 460 | 150 | ~40 |
| 60 | 305 | 200 | 150 |
| 90 | 100 | 220 | 200 |
| 100 | 25 | 235 | 220 |

When a temperature of 235°C was achieved it was maintained for approximately 5 hours and the reaction mixture stirred, the reaction temperature and pressure being monitored at all times. The temperature was then reduced to 180°C and the autoclave vented. The product was removed and the activity of the catalyst assessed as in Example 1. It was not possible to measure the amount of tar formed as in Example 1. Table 2 below shows the amounts of reactants used and the results obtained.

TABLE 2

| POLYISOBUTENE (PIB) | WEIGHT PIB (g) | WEIGHT MALEIC ANHYDRIDE (g) | CATALYST USED | AMOUNT OF CATALYST (ppm) | CATALYST ACTIVITY (%) |
|---|---|---|---|---|---|
| NAPVIS 120 | 2819 | 144.4 | NONE | - | 41.0 |
| NAPVIS 120 | 3007 | 154.2 | DBH | 201 | 47.3 |
| NAPVIS 120 | 2999 | 153.7 | DBTPP | 195 | 56.6 |

DBH and DBTPP have the same meanings as above.

As can be seen from this table when compared with reactions in which no catalyst or a conventional catalyst is used the process of the present invention achieves greater conversion of polyisobutene to polyisobutenyl succinic anhydride. This confirms the efficacy of the present invention.

## Claims

1. A process for the preparation of a polyalkene-substituted succinic acylating agent which comprises reacting a polyalkene and a compound of formula:

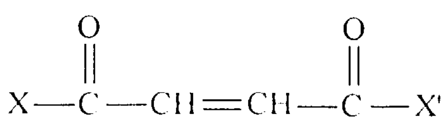

(I)

in which X and X' are the same or different and are each independently selected from -OH, -Cl or -OR" in which R" represents alkyl of 1 to 6 carbon atoms, or X and X' together form a group -O-, characterised in that the reaction takes place in the presence of a dihalotriphenylphosphorane.

2. A process according to claim 1 wherein the reaction is carried out in presence of from 5 to 500ppm dihalotriphenylphosphorane.

3. A process according to claim 1 or 2, wherein the reaction is carried out in presence of from 100 to 400ppm dihalotriphenylphosphorane.

4. A process according to any one of claims 1 to 3, wherein the dihalotriphenylphosphorane is dibromotriphenylphosphorane or dichlorotriphenylphosphorane.

**5.** A process according to claim 4, wherein the dihalotriphenylphosphorane is dibromotriphenylphosphorane.

**6.** A process according to any one of the preceding claims, wherein the polyalkene has a number average molecular weight of 500 to 10,000.

**7.** A process according to claim 6, wherein the polyalkene is polyisobutene.

**8.** A process according to any one of the preceding claims, wherein the compound of formula (I) is maleic acid, maleic anhydride, fumaric acid, or a combination thereof.

**9.** A process according to any one of the preceding claims wherein polyisobutene is reacted with maleic anhydride to obtain polyisobutenyl succinic anhydride.

**10.** A process according to any one of the preceding claims wherein the reaction is carried out at a temperature of from 150 to 300°C.

**11.** A process according to any one of the preceding claims, wherein the molar ratio of polyalkene to compound of formula (I) is from 1:5 to 1:0.8.

**12.** A process for the preparation of an ashless dispersant which comprises preparing a polyalkene-substituted succinic acylating agent by the process defined in any one of claims 1 to 11 and then converting the polyalkene-substituted succinic acylating agent obtained into an ashless dispersant.

**13.** A method of improving the yield of a polyalkene-substituted succinic acylating agent, which comprises preparing said acylating agent by the process defined in any one of claims 1 to 11.

**14.** A method of reducing the amount of undesirable by-products formed in the preparation of a polyalkene-substituted succinic acylating agent, which comprises preparing said acylating agent by the process defined in any one of claims 1 to 11.

**15.** A method of improving the yield, and reducing the amount of undesirable by-products formed, in the preparation of a polyalkene-substituted succinic acylating agent, which comprises preparing said acylating agent by the process defined in any one of claims 1 to 11.

**16.** A method according to claim 14 or 15 wherein the undesirable by-product is tar.

**Revendications**

**1.** Procédé pour la préparation d'un agent d'acylation succinique à substituant polyalcène, qui comprend la réaction d'un polyalcène et d'un composé de formule :

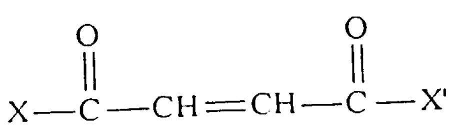

(I)

dans laquelle X et X' sont identiques ou différents et sont choisis chacun indépendamment entre des groupes -OH, -Cl et -OR" dans lequel R" représente un groupe alkyle ayant 1 à 6 atomes de carbone, ou bien X et X', conjointement, forment un groupe -O-, caractérisé en ce que la réaction s'effectue en présence d'un dihalogénotriphénylphosphorane.

**2.** Procédé suivant la revendication 1, dans lequel la réaction est conduite en présence d'une quantité de 5 à 500 ppm d'un dihalogénotriphénylphosphorane.

**3.** Procédé suivant la revendication 1 ou 2, dans lequel la réaction est conduite en présence d'une quantité de 100 à 400 ppm de dihalogénotriphénylphosphorane.

**4.** Procédé suivant l'une quelconque des revendications 1 à 3, dans lequel le dihalogénotriphénylphosphorane est le dibromotriphénylphosphorane ou le dichlorotriphénylphosphorane.

**5.** Procédé suivant la revendication 4, dans lequel le dihalogénotriphénylphosphorane est le dibromotriphénylphosphorane.

**6.** Procédé suivant l'une quelconque des revendications précédentes, dans lequel le polyalcène a une moyenne en nombre du poids moléculaire de 500 à 10 000.

**7.** Procédé suivant la revendication 6, dans lequel le polyalcène consiste en polyisobutène.

**8.** Procédé suivant l'une quelconque des revendications précédentes, dans lequel le composé de formule (I) est l'acide maléique, l'anhydride maléique, l'acide fumarique ou une de leurs associations.

**9.** Procédé suivant l'une quelconque des revendications précédentes, dans lequel le polyisobutène est amené à réagir avec l'anhydride maléique pour obtenir de l'anhydride polyisobuténylsuccinique.

**10.** Procédé suivant l'une quelconque des revendications précédentes, dans lequel la réaction est conduite à une température comprise dans l'intervalle de 150 à 300°C.

**11.** Procédé suivant l'une quelconque des revendications précédentes, dans lequel le rapport molaire du polyalcène au composé de formule (I) est compris dans l'intervalle de 1:5 à 1:0,8.

**12.** Procédé pour la préparation d'un dispersant sans cendres, qui comprend la préparation d'un agent d'acylation succinique à substituant polyalcène par le procédé défini dans l'une quelconque des revendications 1 à 11 et ensuite la conversion de l'agent d'acylation succinique à substituant polyalcène obtenu en un dispersant sans cendres.

**13.** Procédé pour améliorer le rendement en un agent d'acylation succinique à substituant polyalcène, qui comprend la préparation de cet agent d'acylation par le procédé répondant à la définition suivant l'une quelconque des revendications 1 à 11.

**14.** Procédé pour réduire la quantité de sous-produits indésirables formés dans la préparation d'un agent d'acylation succinique à substituant polyalcène, qui comprend la préparation de cet agent d'acylation par le procédé répondant à la définition suivant l'une quelconque des revendications 1 à 11.

**15.** Procédé pour améliorer le rendement, et réduire la quantité de sous-produits indésirables formés, dans la préparation d'un agent d'acylation succinique à substituant polyalcène, qui comprend la préparation de cet agent d'acylation par le procédé répondant à la définition suivant l'une quelconque des revendications 1 à 11.

**16.** Procédé suivant la revendication 14 ou 15, dans lequel le sous-produit indésirable est un goudron.

**Patentansprüche**

**1.** Verfahren zur Herstellung eines Polyalken-substituierten Acylierungsmittels auf der Basis von Bernsteinsäure, umfassend die Reaktion eines Polyalkens und einer Verbindung der Formel

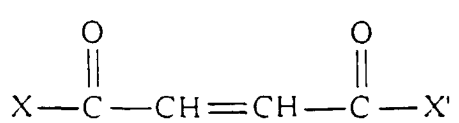

(I)

worin X und X' gleich oder unterschiedlich sind und jeweils unabhängig voneinander ausgewählt werden unter -OH, -Cl oder -OR", worin R" Alkyl mit 1 bis 6 Kohlenstoffatomen bedeutet, oder X und X' zusammen eine -O-Gruppe bilden, dadurch gekennzeichnet, dass die Reaktion in Anwesenheit eines Dihalogentriphenylphosphorans

stattfindet.

2. Verfahren nach Anspruch 1, in dem die Reaktion in Anwesenheit von 5 bis 500 ppm Dihalogentriphenylphosphoran durchgeführt wird.

3. Verfahren nach Anspruch 1 oder 2, in dem die Reaktion in Anwesenheit von 100 bis 400 ppm Dihalogentriphenylphosphoran durchgeführt wird.

4. Verfahren nach einem der Ansprüche 1 bis 3, in dem das Dihalogentriphenylphosphoran Dibromtriphenylphosphoran oder Dichlortriphenylphosphoran ist.

5. Verfahren nach Anspruch 4, in dem das Dihalogentriphenylphosphoran Dibromtriphenylphosphoran ist.

6. Verfahren nach einem der vorstehenden Ansprüche, in dem das Polyalken ein Molekulargewichtszahlenmittel von 500 bis 10.000 besitzt.

7. Verfahren nach Anspruch 6, in dem das Polyalken Polyisobuten ist.

8. Verfahren nach einem der vorstehenden Ansprüche, in dem die Verbindung der Formel (I) Maleinsäure, Maleinsäureanhydrid, Fumarsäure oder eine Kombination davon ist.

9. Verfahren nach einem der vorstehenden Ansprüche, in dem Polyisobuten mit Maleinsäureanhydrid umgesetzt wird, um Polyisobutenylbersteinsäureanhydrid zu erhalten.

10. Verfahren nach einem der vorstehenden Ansprüche, in dem die Reaktion bei einer Temperatur von 150 bis 300 °C durchgeführt wird.

11. Verfahren nach einem der vorstehenden Ansprüche, in dem das Molverhältnis von Polyalken zur Verbindung der Formel (I) 1 : 5 bis 1 : 0,8 beträgt.

12. Verfahren zur Herstellung eines aschefreien Dispergiermittels, umfassend die Herstellung eines Polyalken-substituierten Acylierungsmittels auf der Basis von Bernsteinsäure durch das in einem der Ansprüche 1 bis 11 definierte Verfahren, gefolgt von der Überführung des erhaltenen, Polyalken-substituierten Acylierungsmittels auf Basis von Bernsteinsäure in ein aschefreies Dispergiermittel.

13. Verfahren zur Verbesserung der Ausbeute an Polyalken-substituiertem Acylierungsmittel auf Basis von Bernsteinsäure, umfassend die Herstellung des Acylierungsmittels durch das in einem der Ansprüche 1 bis 11 definierte Verfahren.

14. Verfahren zur Reduzierung der Menge an ungewünschten Nebenprodukten, die bei der Herstellung eines Polyalken-substituierten Acylierungsmittels auf Basis von Bernsteinsäure gebildet werden, umfassend die Herstellung des Acylierungsmittels durch das in einem der Ansprüche 1 bis 11 definierte Verfahren.

15. Verfahren zur Verbesserung der Ausbeute und zur Reduzierung der Menge an ungewünschten Nebenprodukten, die bei der Herstellung eines Polyalken-substituierten Acylierungsmittels auf Basis von Bernsteinsäure gebildet werden, umfassend die Herstellung des Acylierungsmittels durch das in einem der Ansprüche 1 bis 11 definierte Verfahren.

16. Verfahren nach Anspruch 14 oder 15, in dem das ungewünschte Nebenprodukt Teer ist.